# EUROPEAN PATENT SPECIFICATION

(11) **EP 0 478 682 B1**
(45) Date of publication and mention of the grant of the patent: **16.03.1994**
(21) Application number: 90910599.1
(22) Date of filing: 15.06.1990
(51) Int. Cl.: C12Q 1/02, C12Q 1/04, G01N 33/46

(54) **DETECTION OF FUNGAL GROWTH**
NACHWEIS VON SCHIMMELN
DECELEMENT DE LA MOISISSURE

(30) Priority: 15.06.1989 DK 2952/89
(43) Date of publication of application: 08.04.1992
(73) Proprietor: ASSURANCE-COMPAGNIET BALTICA A/S, DK-2750 Ballerup (DK)
(72) Inventor: SUNDBERG, Henrik, DK-2850 Naerum (DK); MUNCH, Ole, DK-6000 Kolding (DK)
(74) Representative: Sundien, Gunnar
(86) International application number: DK9000153
(87) International publication number: WO9015879

(56) References cited:
- US-A- 4 544 546
- DIALOG INFORMATIONAL SERVICE, file 351, World Patent Index; G. VAREKHOV et al., no. 5408875*
- PHYTOPATHOLOGY, vol. 75, no. 3, 1985, J.J. MORELL et al.; pp. 329-332*
- PHYTOPATHOLOGY, vol. 77, no. 12, 1987; C.M. BECKER et al., p. 1746, note 472*
- MYCOLOGIA, vol. 71, 1979; E.L. SCHMID, pp. 627-633*
- JOURNAL PHYTOPATHOLOGY, vol. 123, 1988, U.T. ROONGRUANGSREE et al., pp. 244-252*
- DIALOG INFORMATIONAL SERVICE, file 351, World Patent Index; V.S. BANNIKOW et al., no. 4700300*

## Description

### 1. THE TECHNICAL FIELD

The present invention relates to a method of detecting the viability of fungal cells from fungi infected buildings or building materials after a cell kill treatment.

Detection and assessment of the extent of damage to buildings or building materials in connection with controlling fungal infections may today be performed by various techniques including destructive sampling as well as physically non-destructive measuring methods.

In order to effectively control a fungal infection it is important to know in advance precisely where the fungal attack is located, whether the fungus is alive and if so the required duration and extent of the treatment of the fungus. This is so because in case of older dry fungal infections it is a natural thing that only parts of the fungus are viable, and consequently the repairs of the building may often be limited to the growth zone or may be completely avoided in case of long dead fungi in construction materials with otherwise intact strength properties. Because of the uncertainty related to the viability of the heat treated fungus, the repairs are often extended to include a safety zone, the repair of which further adds to the costs, and may in certain cases be avoided if the viability of the fungus could be ascertained.

Furthermore, it is today difficult to determine at which temperature and for how long a given fungal treatment by means of e.g. a heat treatment must be carried out using e.g. a microwave canon in order to obtain or assist in obtaining efficient and reliable control of the fungus.

When treating and repairing fungal infections, it is therefore important to have at one's disposal a simple method of detecting the viability of fungal cells in buildings or building materials.

### 2. BACKGROUND ART

Until now the method used in practice for detecting the viability of fungal cells from fungi infected buildings or building materials before or after a cell kill treatment has been collecting samples from infected buildings or building materials and transporting the samples to a well equipped biological laboratory, where the samples are grown on suitable substrates. According to known techniques, it is attempted to revive the fungus under optimum conditions, including inoculation on growth substrate and 2-6 months in culturing chamber, whereafter, typically not until after 6 months of unsuccessful reviving attempts, it is considered reasonably likely that the fungus is non-viable.

An example of simple culturing of e.g. dry rot by culturing on substrates containing malt extract and agar is disclosed by S. Cymorek and B. Hegarty, Int., Res. Groups Wood Pres., Doc. No. IRG/WP/2255, 1986.

It is a significant drawback of culturing fungal cells as a test of viability that the method is time consuming. Incubation periods of 2 months or more are often recommended in which period a fungal infection may have grown to an extent which is almost irreparable. It has thus been ascertained that the fungus under favourable conditions may spread at a rate of about 0.5 cm in 24 hours and grow as high as a 4 storage building.

Furthermore, growth of other fungi may often conceal the growth of the fungus which it is desired to detect, i.e. lack of growth of the interesting fungus in the test culturing may both be due to the fungus being dead or to unsuccessful attempts to revive it from its dormant state.

Revival by simple culturing is thus a slow and uncertain method of detecting viability.

Methods for dyeing DNA of the chromosomes are known.

Laurits W. Olson, Opera Botanica, No. 73, 1984, p. 65-66 discloses the Feulgen nucleus dyeing reference method in which the DNA of the chromosomes is dyed by means of basic fuchin.

The techniques of fluorescence microscopy has recently been developed into a valuable tool for biological research for the detection of organisms in biological samples facilitated by nucleic acid staining properties of fluorescent dyes.

US Patent No. 4,544,546 discloses a method of detecting nucleic acids in a biological sample which comprises contacting said biological sample with an effective amount of a dye, thereby producing a complex between the dye and the nucleic acid; irradiating the complex with light of excitation wavelength, thereby causing the complex to emit fluorescence; and measuring the emitted fluorescence. Detection viability of fungal cells from fungi infected buildings or building materials is neither diclosed nor suggested.

World Patent Index, Acc. No. 5408875, G. Varekhov et al. discloses a method of determining the viability of fungi by treating a suspension of mycelium with amino acridine dye and measuring the obtained fluorescence at two wavelengths. Nothing is disclosed or suggested about detecting viability of fungal cells in samples from fungi infected buildings or building materials.

Another fluorecsence based method is the DAPI-nucleus dyeing method according to which the fluorescence dye 4',6-diamino-2-phenylindol, DAPI, is used for dyeing chromosome DNA which subsequently exhibits a blue or bluish colour in the fluorescence microscope. The method has been used for detecting nuclei in spores from dry rot cultivated in laboratory cultures with the purpose of examining the structure and germination of basidiospores, B. Hegarty and U. Schmitt Int. Res. Group. Wood Preservation, 19. Annual Meeting, Madrid, Spain 24-29 April 1988. Use of the DAPI dyeing method in these examinations however does not concern detection of viability within the meaning of the present invention, neither as regards spores nor fungal cells.

### 3. DISCLOSURE OF THE INVENTION

It is the object of the present invention to provide a method of detecting the viability of fungal cells from fungi infected buildings or building materials after a cell kill treatment wherein the risk of detecting false viable cells is reduced.

According to the invention there is provided a method of detecting the viability of fungal cells from fungi infected buildings or building materials after a cell killing treatment wherein a sample from the treated buildings or building materials is subjected to a dyeing treatment with a substance containing a dye used for dyeing the chromosomes in the cell nucleus whereafter the dyed cell nucleus chromosomes are detected, characterized in that the sample is stored for a period long enough for the chromosomes of the killed cells to disappear from the cell nuclei before the dyeing treatment is effected.

Although it is known to dye the DNA, i.e. deoxyribonucleic acid, of the chromosomes in connection with cellular structure examinations and the like, no so-called nucleus dyeing method has until now been provided in connection with detection of the viability of fungal cells from fungi infected buildings or building materials after a cell kill treatment.

From field and laboratory tests performed, it has turned out that the risk of detecting non-viable cells as viable cells can be reduced if the sample is stored for a period long enough for the chromosomes of the killed cells to disappear from the cell nuclei before performing the dyeing treatment.

It is generally believed that the sampled cell material must be fixed immediately after the sampling in order to preserve the sample for later laboratory analysis. If this preservation is not performed, the cells easily become overgrown by other organisms, in particular if the fungal cells are dead, whereby the samples are ruined and the risk of misinterpretation of the viability of the sampled specimens is increased.

By employing a traditional fixation of the sample immediately following a cell killing treatment e.g. a lethal microwave radiation, it has turned out that the chromosomes in the nuclei have not yet disappeared and consequently get fixed "too early". Therefore, in the microscopy, well-preserved nuclei can be detected in cells which in fact are dead. Thus, it has been possible to detect chromosome material in cell nuclei 1 hour after mycelium from actively growing dry rot has been boiled for about 4 minutes. When fixing after 24 hours, the nucleus material has disappeared.

Therefore, according to the invention, the sample from the cell kill treated buildings and building materials after a cell killing treatment is not fixed immediately upon sampling, but it is fixed after a storage period, which ensures that the chromosome material in the nuclei of non-viable cells disappears before fixation, dyeing, and microscoping is effected, whereby the risk of misinterpreting dead cells as viable cells is reduced.

The storage period may advantageously be adjusted to the treatment method of the fungal infection. Thus, in a preferred embodiment particularly in the case of heat exposed fungal myceliae, the storage period can be as long as 48 hours, preferably up to 24 hours; however, shorter periods are possible. Optimum periods are easily adjusted according to the treatment method, the exposure effect and the exposure time.

It has proved to be an advantage to dry, optionally freeze dry, the sampled test material, in particular for samples taken out after heat exposure, whereafter they are kept dry until fixation, dyeing, and microscoping.

Therefore, in a preferred embodiment of the invention, the method is thus characterized in that the sample optionally is pretreated by drying or freeze drying whereafter it is kept dry in a storage period before it is fixed and/or optionally posttreated to separate hyphae.

Before the dyeing treatment, the sample is advatageously fixed. The fixing agent is advantageously selected in view of the treatment method and the test material. In a preferred embodiment the fixing agent perferably comprises ethanol/glacial acetic acid mixtures, particularly in a preferred ratio of 3:1.

The dye used for dyeing the chromosomes in the cell nucleus is a DNA dye, i.e. a substance which dyes DNA, or a fluorescent DNA dye, and the detection is performed by means of light microscopy or fluorescence microscopy, respectively, or by another suitable detection method.

Examples of fluorescence dyes which dye DNA and which are usable for dying chromosomes according to the invention comprise fluorescence dyes, such as bis-amino-phenyl-oxadiazol, acriflavin, pararosanilin, bis-benzimide (Hoechst 33258) and 4',6-diamino-2-phenyl-indol, DAPI. Such dyes have been used for detecting chromosomes and cell nuclei for different purposes.

Further, examples of typical DNA fluorescence dyes are propidium iodide, ethidium bromide, dihydroethidium, ethidium monoazide, 9-azidoacridine, ethidium homodimer (5,5'-diazadecamethylene)bis(3,8-diamino-6-phenylphenanthridium) dichloride, acridine-ethidium heterodimer, acridine homodimer (bis-(6-chloro-2-methoxy-9-acridinyl) spermine), acridine orange, Hoechst 33342, 7-aminoactinomycin D, thiazole orange, 9-amino-6-chloro-2-methoxyacridine, 4,5',8-trimethylpsoralen, and Malachite Green acrylamide. Other useful dyes may be selected by a person skilled in the art.

In a preferred embodiment, the fluorescent DNA dye for dyeing the chromosomes is 4',6-diamino-2-phenylindol, DAPI, in a concentration of preferably 1-5 µg/ml.

In connection with the microscoping, it has further proved to be an advantage to separate the hyphae preferably mechanically in such a way that the test becomes simple and uncomplicated.

Therefore, in a preferred embodiment, the sample is post-treated to separate hyphae, preferably by mechanical pulverization, before being microscoped.

Besides employing a posttreatment of the fixed and dyed sample to separate the hyphae, it has proved to be an advantage to increase the specific DAPI absorption of the chromosome DNA, e.g. in dry rot, by adding a detergent solution. Useful detergent solutions include known detergents preferably Triton 100-X and various detergent concentrations, preferably 5 %.

Therefore, in a preferred embodiment, the cell nucleus chromosome dye containing substance is dissolved in a detergent solution, preferably about 5% of Triton 100-X.

A particularly preferred embodiment is characterized in that
a) the sample optionally is pretreated by drying or freeze drying whereafter it is kept dry in a storage period before it is fixed and/or optionally posttreated to separate hyphae;
b) the optionally predried sample is fixed in an ethanol/glacial acetic acid mixture preferably in the ratio 3:1;
c) the sample is washed in distilled water;
d) the sample is dyed for a period from about 5 min up to about 2 hours preferably with a mixture of 2% of 4',6-diamino-2-phenyl-indol, DAPI, in acetone, and 5% of Triton 100-X detergent having a final DAPI concentration of 1-5 µg/ml;
e) the sample is washed in distilled water;
f) the sample is optionally crushed to separate the individual hyphae; and
f) the sample is microscoped by means of fluorescence microscopy at 1000 x enlargement using a mercury lampe, 365-366 nm, filter, 40-100 x objective and oil immersion.

Within the meaning of the invention the expression "viability" designates the capability of the fungal cells to reproduce themselves so that the fungal cells may propagate and the myceliae spread to the surrounding buildings and building materials.

Further, it is not sufficient that the fungal cells are capable of metabolizing, as fungi under certain conditions, especially of low humidity, may enter a sort of "dormant state", in which their metabolic process act very slowly. In dependence on the kind of fungus the dormant state may last 2-10 years before the fungal cell dies, wherefore the fungal cells are still viable and may be revived under suitable conditions of growth. Viable cells within the meaning of the invention however all comprise a nucleus with chromosomes which enable the cell to reproduce itself. When a fungal cell is no longer viable in the meaning of the invention the chromosomes of the cell nucleus disappear and the cell is no longer capable of reproducing itself.

Fungal cells having a cell nucleus with chromosomes will thus either be viable cells in the form of active cells capable of reproducing themselves or of cells in a dormant state, or be non-viable cells, the chromosomes of which in the nucleus have not yet been dissolved. In the absence of cell nucleus containing chromosomes the fungal cells are no longer viable within the meaning of the invention.

According to the invention fungi include Serpula lacrymans, Coniophora putana, Antrodia sp., Gloeophyllum trabeum, etc. and other fungi capable of infecting buildings and building materials.

According to the invention samples of buildings or building materials include thallus, mycelium, hyphae, unit cells or spores of the fungus.

Buildings or building materials in the meaning of the invention comprise all materials which form part of or may form part of buildings and building constructions which the fungus may infect no matter whether it e.g. means the end of the life of the building material.

### 4. BRIEF DESCRIPTION OF DRAWINGS

In the following the method according to the invention is described in more detail, reference being made to the drawings in which
- fig. 1: shows the presence of cell nucleus chromosome containing fungal hyphae as detected by means of the Feulgen nucleus dyeing method and flourescence microscopy,
- fig. 2: shows the presence of cell nucleus containing viable fungal hyphae in a dormant state as detected by means of the DAPI nucleus dyeing method and fluorescence microscopy according to the invention, and
- fig. 3: shows non-viable fungal hyphae without chromosome containing cell nuclei from mycelium having been boiled for 4 minutes and fixed after 24 hours and measured by means of the DAPI nuclei dyeing method and fluorescence microscopy according to the invention.

### 5. DETAILED DESCRIPTION

A particularly preferred embodiment of the method according to the invention comprises the steps of
1. pretreating the sample by drying, preferably freeze drying, and storing the dried sample in a storage period of 24 hours,
2. fixing the pretreated sample in an ethanol/glacial acetic acid mixture preferably in the ratio 3:1,
3. washing the sample in distilled water,
4. dyeing the sample for about 5 minutes to about 2 hours preferably with a 2% mixture of the fluorescence dye DAPI (Sigma) in an acetone basis solution yielding a final concentration of 1-5µg/ml, and a 5% Triton 100-X detergent solution,
5. washing the sample in distilled water,
6. optionally crushing the sample to separate the individual hyphae by means of a coverglass and a drop of water, and
7. microscoping the sample by means of fluorescence microscopy at 1000 x enlargement using a mercury lampe, 365-366 nm, filter, 40-100 x objective and oil immersion.

### 6. EXAMPLES

The method according to the invention has been used for detecting the viability of fungal cells in both live and dead mycelium from dry rot.

Live myceliae have been cultured non-sterilized from fungi infected wood, stone wool and bridgework, i.e. mortar and bricks, in the laboratory, whereafter they have been tested for sensitivity to the dyes. Dead mycelium has been provided by heat treating the live mycelium in a microwave oven and by boiling for e.g. about 4 to 10 minutes, respectively. Old presumably inactive mycelium has also been provided.

In all cases the method has been satisfactory.

### EXAMPLE 1

To illustrate the method according to the invention the Feulgen nucleus dyeing method has been used on samples of live mycelium from dry rot.

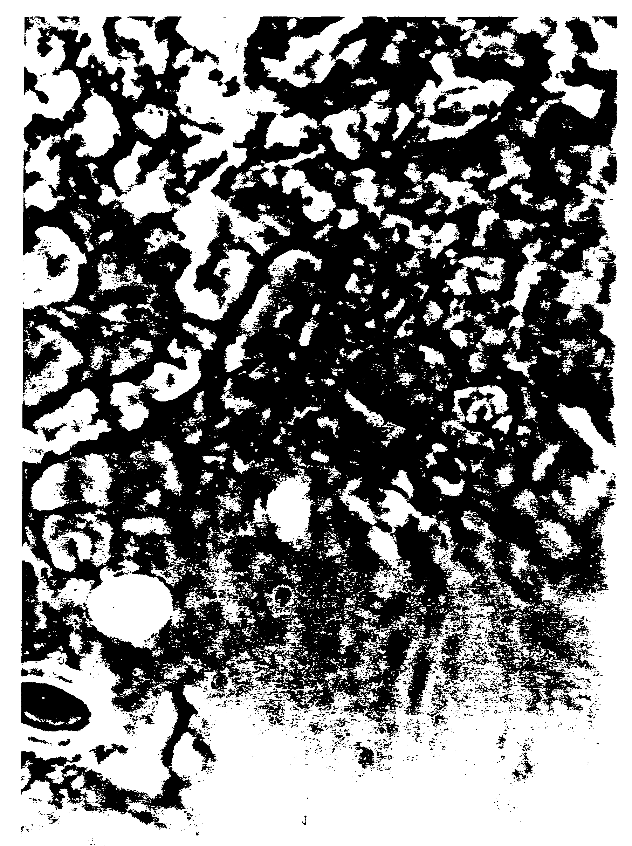

Fig. 1 shows fluorescence microscopy of fungal hyphae from live mycelium of dry rot having been dyed by means of the Feulgen-dyeing method. The arrow in the centre of the picture refers to a nucleus which in a colour picture shows itself as dark red approaching violet on a reddish background. Is appears that the Feulgen dyeing method which usually dyes the chromosomes in the nuclei a bright reddish only dyes fungal myceliae faintly. This is probably due to the chitin content of the cell wall and the scarce amount of chromosomes present in the cell nuclei.

Besides being an extremely time consuming preparation where certain steps in the procedure may easily go wrong the method calls for use of phase contrast microscopy to make possible nuclei detectable. Consequently, the method is less advantageous for use when carrying out the procedure according to the invention.

### EXAMPLE 2

To illustrate the method according to the invention the DAPI nucleus dyeing method has been used on the previously mentioned live and dead mycelium from dry rot.

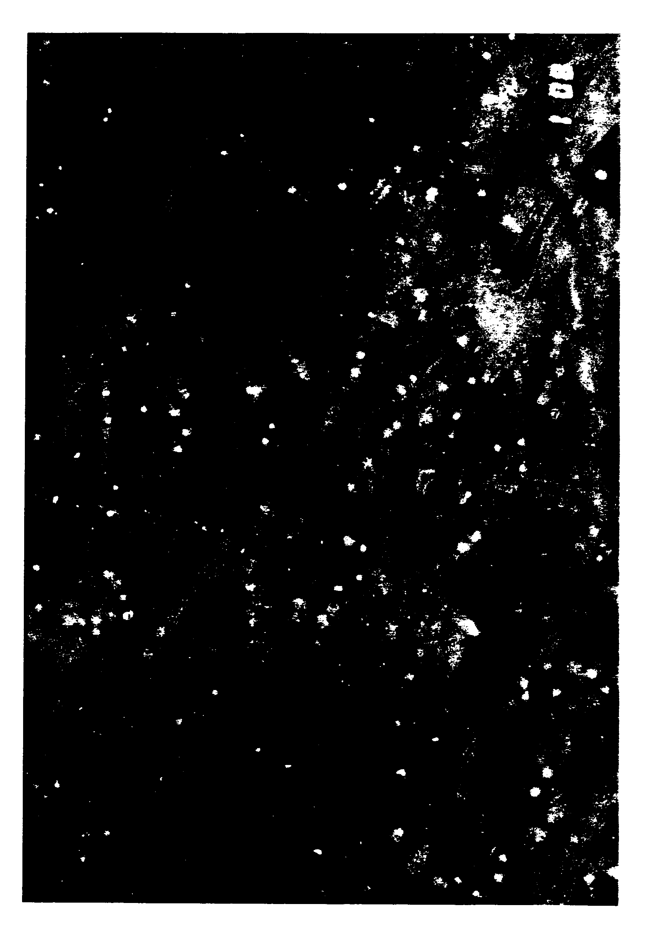

Fig. 2 shows fluorescence microscopy of viable fungal hyphae in a dormant state. In the picture the nuclei dyed bright blue appear as white spots in the more dim contours of the fungal hyphae.

In comparison with the Feulgen nucleus dyeing method the DAPI method is far more practical in use.

### EXAMPLE 3

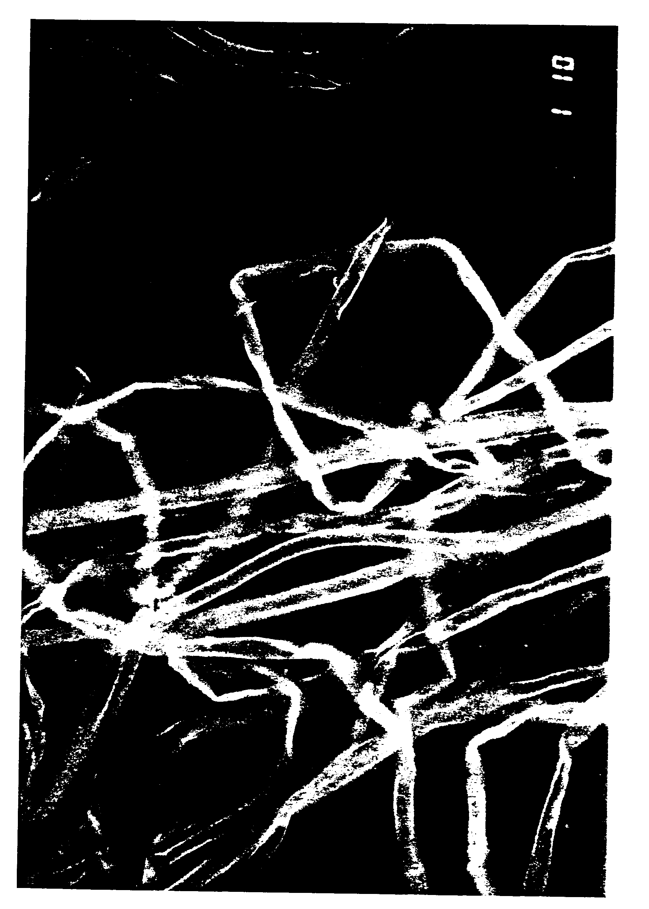

Fig. 3 shows fluorescence microscopy of non-viable fungal hyphae from mycelium having been boiled for 4 minutes and fixed 24 hours later on whereafter they have been dyed by means of the DAPI nucleus dyeing method. The picture clearly shows the absence of dyed nuclei.

## Claims

1. A method of detecting the viability of fungal cells from fungi infected buildings or building materials after a cell killing treatment wherein a sample from the treated buildings or building materials is subjected to a dyeing treatment with a substance containing a dye used for dyeing the chromosomes in the cell nucleus whereafter the dyed cell nucleus chromosomes are detected, **characterized** in that the sample is stored for a period long enough for the chromosomes of the killed cells to disappear from the cell nuclei before the dyeing treatment is effected.

2. A method according to claims 1, **characterized** in that the storage period is up to 48 hours, preferably up to 24 hours, or shorter.

3. A method according to claim 1-2, **characterized** in that the sample is dried before the storage period.

4. A method according to claims 1-3, **characterized** in that the sample is fixed by a fixing agent, preferably a 3:1 ethanol/glacial acetic acid before the dyeing treatment is effected.

5. A method according to the claims 1-4, **characterized** in that after the dyeing treatment the sample is treated to separate hyphae preferably by mechanical pulverization.

6. A method according to claims 1-5, **characterized** in that the dye for dyeing the chromosomes is a fluorescent DNA dye, preferably 4',6-diamino-2-phenyl-indol, DAPI, in a concentration of 1-5 µg/ml.

7. A method according to claims 1-6, **characterized** in that the substance containing the cell nucleus chromosome dye is dissolved in a detergent solution, preferably about 5% Triton® 100-X.

8. A method of detecting the viability of fungal cells from fungi infected buildings or building materials after a cell killing treatment wherein a sample from the treated buildings or the building material is subjected to a dyeing treatment with a substance containing a dye used for dyeing the chromosomes in the cell nucleus whereafter the dyed cell nucleus chromosomes are detected, **characterized** in that
a) the sample optionally is pretreated by drying or freeze drying whereafter it is kept dry in a storage period before it is fixed and/or optionally posttreated to separate hyphae;
b) the optionally predried sample is fixed in an ethanol/glacial acetic acid mixture preferably in the ratio 3:1;
c) the sample is washed in distilled water;
d) the sample is dyed for a period from about 5 minutes up to about 2 hours preferably with a mixture of 2% of 4',6-diamino-2-phenyl-indol, DAPI, in acetone, and 5% of Triton® 100-X detergent having a final DAPI concentration of 1-5 µg/ml;
e) the sample is washed in distilled water;
f) the sample is optionally crushed to separate the individual hyphae; and
g) the sample is microscoped by means of fluorescence microscopy at 1000 x enlargement using a mercury lampe, 365-366 nm, filter, 40-100 x objective and oil immersion.

9. In a method of controlling fungal infection of buildings or building materials, wherein the infected areas are subjected to a cell killing treatment, preferably a heat treatment using microwaves,
samples of the fungi cell kill treated buildings or building materials are dried and thereafter stored for a period long enough for the chromosomes of the killed cells to disappear from the cell nuclei, and in case of heat treatment typically up to 48 hours, preferably up to 24 hours or shorter; and
the stored samples are subjected to a dyeing treatment with a substance containing a dye used for dyeing the chromosomes in the cell nuclei preferably a mixture of 4',6-diamino-2-phenyl-indol, DAPI, and Triton® 100-X whereafter the dyed cell nuclei chromosomes are detected.

## Patentansprüche

1. Ein Verfahren zum Bestimmen der Lebensfähigkeit von Pilzzellen aus pilzinfizierten Gebäuden oder Baumaterialien nach einer Zell-Abtötungsbehandlung, wobei eine Probe von den behandelten Gebäuden oder Baumaterialen einer Anfärbebehandlung mit einer Substanz unterworfen wird, die einen Farbstoff enthält, der zum Anfärben der Chromosomen im Zellkern verwendet wird, worauf die angefärbten Zellkernchromosomen festgestellt werden,
**dadurch gekennzeichnet, daß**
die Probe während einer Zeitdauer abgelagert wird, die lange genug ist, um die Chromosomen der abgetöteten Zellen aus den Zellkernen verschwinden zu lassen, bevor die Anfärbebehandlung ausgeführt wird.

2. Ein Verfahren nach Anspruch 1, dadurch gekennzeichnet, daß die Ablagerungsdauer bis zu 48 Stunden, vorzugsweise bis zu 24 Stunden oder kürzer ist.

3. Ein Verfahren nach Anspruch 1-2, dadurch gekennzeichnet, daß die Probe vor der Ablagerungsperiode getrocknet wird.

4. Ein Verfahren nach Ansprüchen 1-3, dadurch gekennzeichnet, daß die Probe vor Ausführung der Anfärbebehandlung mit einem Fixiermittel, vorzugsweise einer 3:1 Äthanol/Eisessigsäure fixiert wird.

5. Ein Verfahren nach den Ansprüchen 1-4, dadurch gekennzeichnet, daß nach der Anfärbehandlung die Probe behandelt wird, um Hyphen, vorzugsweise durch mechanische Pulverisierung, abzutrennen.

6. Ein Verfahren nach Ansprüchen 1-5, dadurch gekennzeichnet, daß der Farbstoff zum Anfärben der Chromosomen ein fluoreszierender DNA-Farbstoff ist, vorzugsweise ein 4',6-Diamino-2-Phenyl-Indol, DAPI, in einer Konzentration von 1-5 µg/ml.

7. Ein Verfahren nach den Ansprüchen 1-6, dadurch gekennzeichnet, daß die Substanz, welche den Zellkernchromosomenfarbstoff enthält, in einer Detergens-Lösung aufgelöst wird, vorzugsweise etwa 5% Triton® 100-X.

8. Ein Verfahren zum Bestimmen der Lebensfähigkeit von Pilzzellen aus pilzinfizierten Gebäuden oder Baumaterialien nach einer Zell-Abtötungsbehandlung, wobei eine Probe von den behandelten Gebäuden oder dem Baumaterial einer Anfärbebehandlung mit einer Substanz unterworfen wird, die einen Farbstoff enthält, der zum Anfärben der Chromosomen im Zellkern verwendet wird, worauf die angefärbten Zellkernchromosomen festgestellt werden, dadurch gekennzeichnet, daß
a) die Probe wahlweise durch Trocknen oder Gefriertrocknen vorbehandelt wird, worauf sie in einer Ablagerungsperiode trockengehalten wird, bevor sie fixiert und/oder wahlweise zur Abtrennung von Hylphen nachbehandelt wird;
b) die wahlweise vorbehandelte Probe in einer Äthanol/Eisessigsäuremischung, vorzugsweise im Verhältnis 3:1 fixiert wird;
c) die Probe in destilliertem Wasser gewaschen wird;
d) die Probe während einer Zeitdauer von etwa 5 Minuten bis zu etwa 2 Stunden angefärbt wird, vorzugsweise mit einer Mischung aus 2% 4',6-Diamino- Phenyl-Indol, DAPI, in Aceton und 5% Triton® 100-X Detergens mit einer endgültigen DAPI-Konzentration von 1-5 µg/ml;
e) die Probe in destilliertem Wasser gewaschen wird;
f) die Probe wahlweise zerkleinert wird, um die einzelnen Hyphen abzutrennen; und
g) die Probe mit Hilfe der Fluoreszenzmikroskopie bei 1000-facher Vergrößerung mikroskopiert wird, unter Verwendung einer Quecksilberlampe, 365 bis 366 nm, Filter, 40 bis 100 x Ojektiv und Ölimmersion.

9. In einem Verfahren zur Kontrolle der Pilzinfektion von Gebäuden oder Baumaterialien, wobei die infizierten Bereiche einer Zell-Abtötungsbehandlung unterworfen werden, vorzugsweise einer Hitzebehandlung unter Verwendung von Mikrowellen, werden Proben der durch Pilzzellabtötung behandelten Gebäude oder Baumaterialien getrocknet und anschliepend während einer Zeitdauer abgelagert, die lange genug ist, um die Chromosomen der abgetöteten Zellen aus den Zellkernen verschwinden zu lassen, und im Falle einer Wärmebehandlung typischerweise bis zu 48 Stunden, vorzugsweise bis zu 24 Stunden oder kürzer; und werden die abgelagerten Proben einer Anfärbehandlung mit einer Substanz unterworfen, die einen Farbstoff enthält, der zum Anfärben der Chromosomen im Zellkern verwendet wird, vorzugsweise eine Mischung aus 4',6-Diamino-2-Phenyl-Indol, DAPI, und Triton® 100-X, worauf die angefärbten Zellkernchromosomen festgestellt werden.

## Revendications

1. Procédé pour déterminer la viabilité des moisissures cellulaires prélevées sur des bâtiments ou sur des matériaux de construction infectés par des moisissures à la suite d'un traitement destructeur des cellules dans lequel l'on soumet un échantillon prélevé sur les bâtiments ou sur des matériaux de construction décontaminés à un traitement de coloration avec une substance contenant un colorant destiné à colorer les chromosomes à l'intérieur du noyau cellulaire à la suite de quoi les chromosomes en question sont détectés, procédé caractérisé en ce que l'échantillon est conservé pour une durée suffisamment longue pour que les chromosomes des cellules tuées disparaissent du noyau cellulaire avant de procéder au traitement de coloration.

2. Procédé selon la revendication 1, caractérisé en ce que la durée de conservation s'élève à 48 heures, de préférence à 24 heures, ou moins.

3. Procédé selon les revendications 1-2, caractérisé en ce que l'échantillon est séché préalablement à l'étape de conservation.

4. Procédé selon les revendications 1-3, caractérisé en ce que l'échantillon est fixé par un agent fixateur, de préférence un mélange éthanol/ acide acétique glacial 3:1 avant de procéder au traitement de coloration.

5. Procédé de coloration selon les revendications 1-4, caractérisé en ce qu'à la suite du traitement de coloration, l'échantillon est traité de préférence par pulvérisation mécanique afin de séparer les hyphes.

6. Procédé selon les revendications 1-5, caractérisé en ce que le colorant utilisé pour colorer les chromosomes est un colorant fluorescent pour ADN, de préférence le 4',6-diamino-2-phényl-indole, à raison de 1-5 µg / ml.

7. Procédé selon les revendications 1-6, caractérisé en ce que la substance contenant le colorant destiné à colorer les chromosomes du noyau cellulaire est dissoute dans une solution détergente, de préférence dans du Triton 100-X à 5 % environ.

8. Procédé pour déterminer la viabilité des moisissures cellulaires prélevées sur des bâtiments ou sur des matériaux de construction infectés par des moisissures à la suite d'un traitement destructeur des cellules dans lequel l'on soumet un échantillon prélevé sur des bâtiments ou sur les matériaux de construction décontaminés à un traitement de coloration avec une substance contenant un colorant destiné à colorer les chromosomes à l'intérieur du noyau cellulaire à la suite de quoi les chromosomes colorés en question sont détectés, procédé caractérisé en ce que
a) l'échantillon est soumis, le cas échéant, à un prétraitement de séchage ou de lyophilisation à la suite de quoi il est conservé à l'état sec pour une certaine durée avant d'être fixé et/ ou traité ultérieurement de manière facultative afin de séparer les hyphes.
b) l'échantillon éventuellement précédé est fixé au préalable avec un mélange éthanol/ acide acétique glacial de préférence en proportion de 3:1;
c) I'échantillon est lavé avec de l'eau distillée;
d) I'échantillon est coloré pendant un temps allant d'environ 5 minutes à environ 2 heures de préférence avec un mélange de 4',6-diamino-2-phényl-indole, DAPI, 2%, dans de l'acétone, et de détergent Triton® 100-X à 5 % dont la concentration finale en DAPI est de 1-5 µg/ml;
e) I'échantillon est lavé avec de l'eau distillée;
f) I'échantillon est facultativement broyé afin de séparer les différents hyphes; et
g) I'échantillon est examiné au microscope à fluorescence à un grossissement de 1000 X en employant une lampe à mercure, 365-366 nm, objectif 40-100 X et sous immersion d'huile.

9. Procédé pour lutter contre l'infection des bâtiments ou des matériaux de construction par des moisissures, dans lequel les parties infectées sont soumises à un traitement destructeur des cellules, de préférence à un traitement thermique au moyen de micro-ondes, procédé dans lequel les échantillons de bâtiments ou de matériaux de construction décontaminés sont séchés et conservés par la suite pour une durée suffisamment longue pouvant aller communément jusqu'à 48 heures dans le cas de traitement thermique, de préférence jusqu'à 24 heures ou moins, afin que les chromosomes des cellules tuées disparaissent, et dans lequel les échantillons conservés sont soumis à un traitement de coloration avec une substance contenant un colorant utilisé pour colorer les chromosomes à l'intérieur du noyau cellulaire, de préférence un mélange de 4', 6-diamino-2-phényl-indole, DAPI, et de Triton® 100-X à la suite de quoi les chromosomes colorés du noyau cellulaire sont détectés.
